Europäisches Patentamt

European Patent Office

Office européen des brevets

(19)

(11) Veröffentlichungsnummer: **0 230 006 B1**

(12) # EUROPÄISCHE PATENTSCHRIFT

(45) Veröffentlichungstag der Patentschrift:
04.04.90

(51) Int. Cl.⁴: **A61F 2/30**

(21) Anmeldenummer: **86117541.2**

(22) Anmeldetag: **17.12.86**

(54) Knochenimplantat.

(30) Priorität: **16.01.86 DE 8600970 U**

(43) Veröffentlichungstag der Anmeldung:
**29.07.87 Patentblatt 87/31**

(45) Bekanntmachung des Hinweises auf die Patenterteilung:
**04.04.90 Patentblatt 90/14**

(84) Benannte Vertragsstaaten:
**DE ES FR GB IT SE**

(56) Entgegenhaltungen:
**CH-A- 611 794**
**FR-A- 2 315 902**
**GB-A- 2 024 631**
**GB-A- 2 059 267**

(73) Patentinhaber: **Waldemar Link GmbH & Co,
Barkhausenweg 10, D-2000 Hamburg 63(DE)**

(72) Erfinder: **Keller, Arnold, An der Naherfurth 5,
D-2061 Kayhude(DE)**

(74) Vertreter: **Glawe, Delfs, Moll & Partner Patentanwälte,
Postfach 26 01 62 Liebherrstrasse 20,
D-8000 München 26(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Knochenimplantat mit einer eine Vielzahl von Durchbrechungen zum Einwachsen von Knochensubstanz aufweisenden, netzartigen Oberflächenabdeckung, die von einander kreuzenden Scharen von langgestreckten Elementen gebildet ist.

Endoprothesen für die zementfreie Knochenimplantation werden häufig mit einer Oberflächenstruktur ausgerüstet, die zwecks besserer Kraftübertragung Vertiefungen oder Durchbrechungen aufweist zum Einwachsen von Knochenmaterial. Bekannt ist es beispielsweise, die Implantatoberfläche mit sog. Rippenstreckmetall (DE-OS 34 25 002) oder Metallgewebe (EP-OS 0 038 902) zu belegen. Dies geschieht im Herstellungsverfahren durch Belegen des Implantatmodells mit den in Wachs nachgebildeten Oberflächenstrukturen, wobei sich im anschließenden Gußverfahren die Oberflächenstruktur einstückig mit dem Kernmaterial herausbildet. Da dabei das Modell der Oberflächenstruktur unmittelbar auf das Modell des Kernimplantats aufgelegt wird, wird die Form der zwischen der Oberflächenabdeckung und der Implantatoberfläche für die Anlagerung von Knochenmaterial zur Verfügung stehenden freien Räume durch die Form der Abdeckung selbst bestimmt. Bei der Betrachtung der Form dieser Räume bei bekannten Oberflächenabdeckungen ergibt sich, daß diese verhältnismäßig ungünstig ist, weil die diese Abdeckung bildenden langgestreckten Elemente mit einem großen Teil ihrer Länge an der Implantatoberfläche anliegen und sich zwischen den Anlagebereichen nur kleine Öffnungen mit spitz zulaufenden Endzwickeln ergeben, die nur das Einwachsen von verhältnismäßig schwachen Knochenbrücken erlauben und damit die Größe der quer zur Implantatoberfläche übertragbaren Zugkräfte beschränken. Außerdem haben die die Oberflächenabdeckung bildenden langgestreckten Elemente eine verhältnismäßig große Dicke im Verhältnis zur Weite der zwischen ihnen und der Implantatoberfläche belassenen Räume, so daß diese Räume im Knochenanlagerungsprozeß erst verhältnismäßig spät erreicht werden und damit der Prozeß des "Einwachsens" der Prothese in das natürliche Knochengewebe verzögert wird.

Der Erfindung liegt die Aufgabe zugrunde, ein Knochenimplantat der eingangs genannten Art zu schaffen, das günstigere Voraussetzungen für die Kraftübertragung bei zementfreier Implantation und ein rasches Einwachsen der Knochensubstanz ermöglicht.

Die erfindungsgemäße Lösung besteht darin,, daß die langgestreckten Elemente wenigstens einer der beiden Scharen einen im wesentlichen konstanten Abstand von der darunter liegenden Implantatoberfläche aufweisen.

Dies ergibt die Möglichkeit, die Weite der zwischen der Oberflächenabdeckung und der Implantatoberfläche befindlichen Räume beliebig groß zu gestalten.

Zweckmäßigerweise haben die langgestreckten Elemente beider Scharen einen im wesentlichen konstanten Abstand von der darunter liegenden Implantatoberfläche, wobei Vorsprünge zur distanzhaltenden Verbindung der Abdeckung mit der Oberfläche vorgesehen sind. Jedoch ist es statt dessen auch möglich, daß die langgestreckten Elemente einer der beiden Scharen ganz oder teilweise direkt auf der Implantatoberfläche aufliegen, während lediglich die langgestreckten Elemente der anderen Schar von der Implantatoberfläche mit konstantem Abstand abgehoben sind.

Die Verbindungsvorsprünge können an den langgestreckten Elementen jeweils einer der beiden Scharen vorgesehen sein. Sie können in einer Oberflächenrichtung länglich ausgebildet sein. Dadurch erhalten sie rippen- oder flächenartigen Charakter, der es ihnen ermöglicht, Kräfte in ihrer Querrichtung in höherem Maße als in ihrer Längsrichtung aufzunehmen. Dies kann erwünscht sein, wenn vornehmlich Kräfte einer bestimmten Richtung zu übertragen sind.

Ferner kann nach der Erfindung vorgesehen sein, daß die langgestreckten Elemente der anderen der beiden Scharen nach außen vorragende Vorsprünge tragen, die in einer quer zu den nach innen vorragenden Vorsprüngen verlaufenden Oberflächenrichtung länglich sind, um einer erhöhte Kraftübertragung in einer anderen Richtung zu ermöglichen. Besonders vorteilhaft ist es, wenn die nach außen vorragenden Vorsprünge quer zu derjenigen Richtung verlaufen, in welcher die Prothese in den Knochen eingefügt wird, um durch Abschaben von Knochenmaterial, das sich in den Zwischenräumen sammelt, günstigere Voraussetzungen für die Füllung der Zwischenräume mit neugebildeter Knochensubstanz zu schaffen, wie dies an sich bekannt ist.

Zweckmäßigerweise haben die langgestreckten Elemente beider Scharen im wesentlichen gleichen Abstand von der darunter liegenden Oberfläche, obwohl sie je nach Art und Hauptrichtung der zu übertragenden Kräfte auch unterschiedlich gelegen sein können. Sie sind zweckmäßigerweise mit im wesentlichen konstantem Querschnitt ausgeführt, wenn man von den von ihnen ausgehenden Vorsprüngen absieht, und nehmen damit den Charakter von Stäbchen oder Drähten an, zumal wenn sie nach einem weiteren Merkmal der Erfindung im wesentlichen kreisförmig begrenzten Querschnitt haben.

Die Vorsprünge sind zweckmäßigerweise an den Kreuzungspunkten der langgestreckten Elemente vorgesehen, um die Abdeckung in diesen besonders beanspruchten Bereichen zu stabilisieren. Das gilt sowohl für den fragilen Modellzustand der Abdeckungen als auch für das Endprodukt.

Die die Struktur der Abdeckung charakterisierenden Maße liegen zweckmäßigerweise in Millimetergrößenordnung, nämlich der lichte gegenseitige Abstand der langgestreckten Elemente einer Schar zwischen 0,5 und 4mm (vorzugsweise 1 und 3mm), die Dicke der langgestreckten Elemente vorzugsweise zwischen 0,5 und 2mm (insbesondere zwischen 0,7 und 1,5mm) und der lichte Abstand der langgestreckten Elemente von der darunter liegenden Oberfläche zwischen 0,5 und 1,5mm. Das Verhältnis des lichten Abstands der langgestreckten Elemente voneinander zu ihrer Dicke liegt zweckmäßigerweise zwischen 1 und 4mm (insbesondere zwischen 2 und 3mm).

Die erfindungsgemäße Oberflächenabdeckung kann sowohl an Prothesenschäften als auch an flächigen Prothesenteilen, wie zum Beispiel an Kniegelenkschalenprothesen, angebracht werden. Sie ermöglicht einen verhältnismäßg genau definierten Abstand sowohl zur Oberfläche des Prothesenkernmaterials hin als auch gegenüber dem Knochen, so daß sich für die sich neu bildenden Knochenstrukturen ein gewisser und gewünschter Zwischenraum darstellt.

Die Erfindung wird im folgenden näher unter Bezugnahme auf das in der Zeichnung dargestellte Ausführungsbeispiel erläutert.
Darin zeigen:

Fig. 1 eine Seitenansicht einer Hüftgelenk-Schaftprothese und
Fig. 2 eine vergrößerte, perspektivische Darstellung der Oberflächenabdeckung.

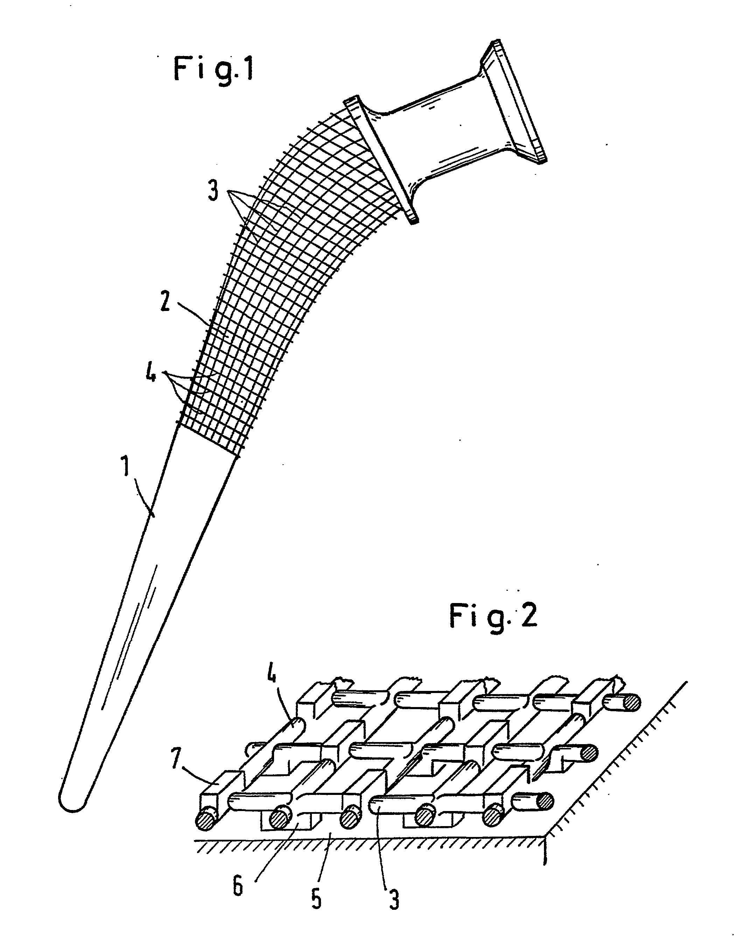

Der Prothesenschaft 1 der in Fig. 1 dargestellten Hüftgelenkprothese ist in seinem proximalen Abschnitt ringsum belegt mit einem Netz oder Gitter 2, das von in Längsrichtung und in Querrichtung verlaufenden Drähten 3 bzw. 4 gebildet ist.

Wie man der Darstellung in Fig. 2 entnimmt, liegen die Längs- und Querdrähte 3 bzw. 4 in derselben Ebene und in gleichem Abstand über der Oberfläche 5 des Implantatkerns. Dieser Abstand wird gesichert durch Vorsprünge 6, die von den Längsdrähten 3 nach innen vorragen und an den Kreuzungspunkten mit den Querdrähten 4 angeordnet sind. Sie sind langgestreckt in Längsrichtung ausgebildet, nämlich etwa dreimal so lang wie dick, wobei der Längsabstand zweier miteinander fluchtender Vorsprünge mindestens ebensogroß ist wie deren Einzellänge. Sie sind an jedem zweiten Kreuzungspunkt der Längs- und Querdrähte angeordnet und bewirken daher an diesen Stellen eine Verstärkung.

An den nicht von den inneren Vorsprüngen besetzten Kreuzungspunkten der Längs- und Querdrähte befinden sich nach außen ragende Vorsprünge 7 gleicher Gestalt und Anordnung aber mit querverlaufender Längsrichtung.

Bei praktischer Erprobung bewährten sich folgene Maße:
Dicke der Längs- und Querdrähte 1mm; lichter Abstand benachbarter Drähte 2,5mm; ursprünglich rechtwinklige Anordnung mit Verformung bei Anpassung an den Implantatkern; Länge/Dicke/Höhe der Vorsprünge 3/1/1mm; Längsabstand der Vorsprünge 4mm.

**Patentansprüche**

1. Knochenimplantat mit einer eine Vielzahl von Durchbrechungen zum Einwachsen von Knochensubstanz aufweisenden, netzartigen Oberflächenabdeckung, die von wenigstens zwei einander kreuzenden Scharen von langgestreckten Elementen (3, 4) gebildet ist, dadurch gekennzeichnet, daß die langgestreckten Elemente (3,4) wenigstens einer der beiden Scharen einen im wesentlichen konstanten Abstand von der darunter liegenden Implantatoberfläche (5) aufweisen.

2. Knochenimplantat nach Anspruch 1, dadurch gekennzeichnet, daß die langgestreckten Elemente (3,4) beider Scharen einen im wesentlichen konstanten Abstand von der darunter liegenden Implantatoberfläche (5) aufweisen und Vorsprünge (6) zur distanzhaltenden Verbindung der Abdeckung mit dieser Oberfläche vorgesehen sind.

3. Knochenimplantat nach Anspruch 2, dadurch gekennzeichnet, daß die langgestreckten Elemente (3,4) einer der beiden Scharen die Verbindungsvorsprünge (6) tragen.

4. Knochenimplantat nach Anspruch 3, dadurch gekennzeichnet, daß die Verbindungsvorsprünge (6) in einer Oberflächenrichtung länglich ausgebildet sind.

5. Knochenimplantat nach Anspruch 4, dadurch gekennzeichnet, daß die langgestreckten Elemente (3,4) der anderen der beiden Scharen nach außen vorragende Vorsprünge (7) tragen, die in einer quer zu den nach innen vorragenden Vorsprüngen verlaufenden Oberflächenrichtung länglich sind.

6. Knochenimplantat nach einem der Ansprüche 1 bis 5, dadurch gekennzeichnet, daß die langgestreckten Elemente (3,4) beider Scharen gleichen Abstand von der darunter liegenden Oberfläche (5) aufweisen.

7. Knochenimplantat nach einem der Ansprüche 1 bis 6, dadurch gekennzeichnet, daß die langgestreckten Elemente (3,4) - abgesehen von den Vorsprüngen (6,7) - im wesentlichen konstanten Querschnitt besitzen.

8. Knochenimplantat von einem der Ansprüche 1 bis 7, dadurch gekennzeichnet, daß die langgestreckten Elemente (3,4) einen im wesentlichen kreisförmig begrenzten Querschnitt aufweisen.

9. Knochenimplantat nach einem der Ansprüche 1 bis 8, dadurch gekennzeichnet, daß die langgestreckten Elemente (3,4) jeder Schar einen gegenseitigen lichten Abstand von 0,5 bis 4mm aufweisen.

10. Knochenimplantat nach Anspruch 9, dadurch gekennzeichnet, daß die langgestreckten Elemente (3,4) einen gegenseitigen Abstand von 1 bis 3mm aufweisen.

11. Knochenimplantat nach einem der Ansprüche 1 bis 10, dadurch gekennzeichnet, daß die Dicke der langgestreckten Elemente (3,4) 0,5 bis 2mm beträgt.

12. Knochenimplantat nach einem der Ansprüche 1 bis 11, dadurch gekennzeichnet, daß das Verhältnis des lichten Abstands der langgestreckten Elemente zu ihrer Dicke zwischen 1 und 4 liegt.

13. Knochenimplantat nach Anspruch 12, dadurch gekennzeichnet, daß das Verhältnis zwischen 2 und 3 liegt.

14. Knochenimplantat nach einem der Ansprüche 1 bis 13, dadurch gekennzeichnet, daß der lichte Abstand der langgestreckten Elemente zu der darunter liegenden Oberfläche (5) zwischen etwa 0,5 und 1,5mm liegt.

15. Knochenimplantat nach einem der Ansprüche 2 bis 5, dadurch gekennzeichnet, daß die Vorsprünge (6,7) an den Kreuzungspunkten der langgestreckten Elemente (3,4) vorgesehen sind.

16. Knochenimplantat nach Anspruch 5, dadurch gekennzeichnet, daß die nach außen vorragenden Vorsprünge (7) quer zur Einfügungsrichtung des Implantats verlaufen.

**Claims**

1. A bone implant with a net-like surface covering which has a plurality of openings for the growth of bone matter and which is formed by at least two mutually intersecting sets of elongate elements (3, 4), characterised in that the elongate elements (3, 4) of at least one of the two sets are at a substantially constant distance from the implant surface (5) disposed thereunder.

2. A bone implant according to claim 1, characterised in that the elongate elements (3, 4) of both sets are at a substantially constant distance from the implant surface (5) disposed thereunder and projections (6) are provided for the spaced connection of the covering to this surface.

3. A bone implant according to claim 2, characterised in that the elongate elements (3, 4) of one of the two sets include the connecting projections (6).

4. A bone implant according to claim 3, characterised in that the connecting projections (6) are of oblong shape in a surface direction.

5. A bone implant according to claim 4, , characterised in that the elongate elements (3, 4) of the other of the two sets have outwardly protruding projections (7) which are oblong in a surface direction extending transversely to the inwardly protruding projections.

6. A bone implant according to any one of claims 1 to 5, characterised in that the elongate elements (3, 4) of both sets are at the same distance from the surface (5) disposed thereunder.

7. A bone implant according to any one of claims 1 to 6, characterised in that the elongate elements (3, 4), apart from the projections (6, 7), are of substantially constant cross-section.

8. A bone implant according to any one of claims 1 to 7, characterised in that the elongate elements (3, 4) are of a cross-section substantially bounded by a circle.

9. A bone implant according to any one of claims 1 to 8, characterised in that the elongate elements (3, 4) of each set have a mutual unobstructed spacing of from 0.5 to 4 mm.

10. A bone implant according to claim 9, characterised in that the elongate elements (3, 4) have a mutual spacing of from 1 to 3 mm.

11. A bone implant according to any one of claims 1 to 10, characterised in that the thickness of the elongate elements (3, 4) is 0.5 to 2 mm.

12. A bone implant according to any one of claims 1 to 11, characterised in that the ratio of the unobstructed spacing of the elongate elements to their thickness is between 1 and 4.

13. A bone implant according to claim 12, characterised in that said ratio is between 2 and 3.

14. A bone implant according to any one of claims 1 to 13, characterised in that the unobstructed spacing of the elongate elements from the surface (5) disposed thereunder is between 0.5 and 1.5 mm.

15. A bone implant according to any one of claims 2 to 5, characterised in that the projections (6, 7) are provided at the points of intersection of the elongate elements (3, 4).

16. A bone implant according to claim 5, characterised in that the outwardly protruding projections (7) extend transversely to the insertion direction of the implant.

**Revendications**

1. Implant osseux comportant un revêtement superficiel réticulé qui présente une multiplicité de creux pour l'implantation de substance osseuse et qui est formé par au moins deux séries d'éléments de forme allongée (3, 4) qui se croisent mutuellement, caractérisé en ce que les éléments de forme allongée (3, 4) de l'une au moins des deux séries sont séparés par une distance pratiquement constante de la surface (5) de l'implant située au-dessous.

2. Implant osseux selon la revendication 1, caractérisé en ce que les éléments de forme allongée (3, 4) des deux séries sont séparés par une distance pratiquement constante de la surface (5) de l'implant située au-dessous et en ce que des saillies (6) sont prévues pour joindre le revêtement à cette surface en maintenant ladite distance.

3. Implant osseux selon la revendication 2, caractérisé en ce que les éléments de forme allongée (3, 4) de l'une des séries portent les saillies de jonction (6).

4. Implant osseux selon la revendication 3, caractérisé en ce que les saillies de jonction (6) sont réalisées avec une forme oblongue dans une direction de la surface.

5. Implant osseux selon la revendication 4, caractérisé en ce que les éléments de forme allongée (3, 4) de l'autre des deux séries portent des saillies (7) en relief vers l'extérieur qui sont oblongues dans une direction de la surface qui s'étend perpendiculairement aux saillies en relief vers l'intérieur.

6. Implant osseux selon l'une quelconque des revendications 1 à 5, caractérisé en ce que les éléments de forme allongée (3, 4) des deux séries sont séparés par la même distance de la surface (5) située au-dessous.

7. Implant osseux selon l'une quelconque des revendications 1 à 6, caractérisé en ce qu'abstraction faite des saillies (6, 7), les éléments de forme allongée (3, 4) ont une section transversale pratiquement constante.

8. Implant osseux selon l'une quelconque des revendications 1 à 7, caractérisé en ce que les éléments de forme allongée (3, 4) présentent une section transversale essentiellement circulaire.

9. Implant osseux selon l'une quelconque des revendications 1 à 8, caractérisé en ce que les éléments de forme allongée (3, 4) de chaque série sont séparés par une distance mutuelle libre de 0,5 à 4 mm.

10. Implant osseux selon la revendication 9, caractérisé en ce que les éléments de forme allongée (3, 4) sont séparés par une distance mutuelle de 1 à 3 mm.

11. Implant osseux selon l'une quelconque des revendications 1 à 10, caractérisé en ce que l'épaisseur des éléments de forme allongée (3, 4) s'élève à 0,5–2 mm.

12. Implant osseux selon l'une quelconque des revendications 1 à 11, caractérisé en ce que le rapport de la distance libre entre les éléments de forme allongée à leur épaisseur se situe entre 1 et 4.

13. Implant osseux selon la revendication 12, caractérisé en ce que le rapport se situe entre 2 et 3.

14. Implant osseux selon l'une quelconque des revendications 1 à 13, caractérisé en ce que la distance libre entre les éléments de forme allongée et la surface (5) située au-dessous se situe entre 0,5 et 1,5 mm environ.

15. Implant osseux selon l'une quelconque des revendications 2 à 5, caractérisé en ce que les saillies (6, 7) sont prévues aux points d'intersection des éléments de forme allongée (3, 4).

16. Implant osseux selon la revendication 5, caractérisé en ce que les saillies (7) en relief vers l'extérieur s'étendent perpendiculairement à la direction d'insertion de l'implant.

Fig.1

3

2

4

1

Fig.2

4

7

6

5

3